# EUROPÄISCHE PATENTANMELDUNG

(11) **EP 3 834 783 A1**
(43) Veröffentlichungstag der Anmeldung: **16.06.2021**
(21) Anmeldenummer: 19216014.1
(22) Anmeldetag: 13.12.2019
(51) Int. Cl.: A61F 5/01

(54) **PLATTE ZUM EINSETZEN IN DEN GAUMEN SOWIE EIN VERFAHREN ZU DEREN HERSTELLUNG**

(71) Anmelder: Matuschek, Carsten, 14612 Falkensee (DE)
(72) Erfinder: Matuschek, Carsten, 14612 Falkensee (DE)
(74) Vertreter: Pfenning, Meinig & Partner mbB

(57) **Zusammenfassung**

Die Erfindung bezieht sich auf eine Platte (1) zum Einsetzen in den Gaumen, wobei die Platte (1) beiderseits einer senkrecht zur Platte (1) gedachten Mittelebene (9) verlaufende Flügel (4) aufweist, die derart ausgebildet sind, um wenigstens bereichsweise am Gaumen anzuliegen. Die zwei Stege (3) verlaufen beiderseits der Mittelebene (9), so dass ein zwischen den Stegen (3) verlaufender Freiraum (6) zum Aufnehmen des Vomers (13) ausgebildet ist.

## Beschreibung

Gegenstand der vorliegenden Anmeldung ist eine Platte zum Einsetzen in den Gaumen mit den Merkmalen des Oberbegriffs des Anspruchs 1, ein Satz von Platten nach Anspruch 12 sowie ein Verfahren zum Herstellen einer Platte zum Einsetzen in den Gaumen nach Anspruch 14.

Es ist bekannt, dass eine Lippen-Kiefer-Gaumen-Segelspalte meist durch eine fehlentwickelte oder -positionierte Prämaxilla begründet ist. Bekanntermaßen tritt dabei ebenfalls das Vomer in einem gebogenen oder verkrümmten Zustand auf. Bleibt eine derartig auftretende Fehlentwicklung oder -stellung einer Lippen-Kiefer-Gaumen-Segelspalte unbehandelt, so kann eine Obstruktion der nasalen Luftwege auftreten, die dazu führt, dass die Betroffenen nur noch einseitig atmen können bzw. mit einem erheblichen Atmungsdefizit leben oder sich zur Behebung der nasalen Beeinträchtigung einem operativen Eingriff unterziehen müssen.

Eine Lippen-Kiefer-Gaumen-Segelspalte liegt üblicherweise vor, wenn eine durchgehende Spalte in Oberlippe, Oberkiefer und Gaumen vorhanden ist. Der Begriff Lippen-Kiefer-Gaumen-Segelspalte soll in dieser Anmeldung allerdings auch für Spaltformen dienen, bei denen beispielsweise nur eine oder zwei dieser Strukturen betroffen sind. Beispiele hierfür sind Lippenspalte, Lippen-Kieferspalte oder Gaumenspalte.

Im Stand der Technik sind Vorrichtungen und Verfahren zur Behandlung der auftretenden Fehlstellungen bei einer Lippen-Kiefer-Gaumen-Segelspalte bekannt. In diesen Verfahren ist es vorgesehen, die Position der Prämaxilla und des Vomers des Säuglings in den ersten Tagen nach der Geburt eines Säuglings mit Lippen-Kiefer-Gaumen-Segelspalte durch Verwendung einer Gaumenplatte ohne operativen Eingriff zu bewegen bzw. zu verschieben. Im Allgemeinen erfolgt eine derartige Behandlung mit der Gaumenplatte in den ersten Tagen des Säuglings. Denn bereits wenige Tage nach der Geburt beginnt der Oberkiefer des Säuglings zu verwachsen und schon wenige Wochen nach der Geburt ist eine Verschiebung der Prämaxilla und des Vomers durch eine Gaumenplatte so gut wie unmöglich.

Als eine derartige herausnehmbare Gaumenplatte ist beispielsweise die Platte nach Hotz (T. Fuchigami et al., Comparison of short-term effects of presurgical nasoalveolar molding and Hotz's plate on maxillary arch form in unilateral cleft lip and palate, Journal of Oral and Maxillofacial Surgery, Medicine, and Pathology, Volume 31, Issue 1, January 2019, Pages 25-30) bekannt. Diese individuell angefertigte Platte kann in den ersten Lebenstagen in den Mundraum des Säuglings eingesetzt werden, um das Wachstum der Oberkiefersegmente zu steuern. Durch die eingesetzte Platte nach Hotz wird der Unterkiefer des Säuglings aus der Ruhelage herausgenommen, sodass ein therapeutisches Muskelungleichgewicht erzeugt wird. Je nach Ausgestaltung der Platte können so gezielt Wachstumsreize oder Wachstumsblockaden stimuliert werden.

Ein weiteres bekanntes Beispiel aus dem Stand der Technik ist die Methode des Nasoalveolar Molding (NAM) nach Grayson (Grayson BH, Shetye PR, Presurgical nasoalveolar moulding treatment in cleft lip and palate patients, Indian J Plast. Surg., October 2009, 42 Suppl., S56-S61). Bei dieser Methode wird eine herausnehmbare Gaumenplatte gemäß einem Abdruck des Oberkiefers des Säuglings individuell angefertigt. Durch Einsetzen der NAM-Gaumenplatte in den Mundraum des Säuglings werden Fehlstellungen der Prämaxilla durch Krafteinwirkung verringert. Zusätzlich besitzt die Gaumenplatte nach Grayson extraoral vorstehende Stützen, wodurch neben der Korrektur der Prämaxilla ebenfalls eine Korrektur von Nasenknorpeldeformitäten ermöglicht wird. Dabei wird eine zusätzliche Verbiegung des Vomers zugelassen. Eine Korrektur oder Begradigung der Form des Vomers ist allerdings bei der Behandlung mit einer NAM-Gaumenplatte nicht möglich.

Es hat sich jedoch gezeigt, dass die Behandlung mit den bekannten Gaumenplatten die Fehlstellung der Prämaxilla eines Säuglings mit Lippen-Kiefer-Gaumen-Segelspalte zwar verringern kann, jedoch eine Begradigung des Vomers oder eine Vermeidung der Krümmung des Vomers nicht oder wenigstens nicht immer in ausreichendem Maße erreicht wird.

Daher ist es die Aufgabe der vorliegenden Erfindung eine kieferorthopädische Apparatur zur Behandlung einer Lippen-Kiefer-Gaumen-Segelspalte zu entwickeln, die die Lebensqualität des Patienten verbessern kann.

Diese Aufgabe wird durch eine Platte zum Einsetzen in den Gaumen und durch ein Verfahren zum Herstellen einer Platte zum Einsetzen in den Gaumen gemäß den unabhängigen Ansprüchen sowie durch einen Satz von Platten zum sequenziellen Begradigen des Vomers eines Säuglings mit Lippen-Kiefer-Gaumen-Segelspalte gelöst. Spezielle Ausführungsformen sind in der Beschreibung, in den Unteransprüchen sowie durch die Figuren beschrieben.

Die hier vorgeschlagene Platte zum Einsetzen in den Gaumen weist beiderseits einer senkrecht zur Platte gedachte Mittelebene verlaufende Flügel auf, die derart ausgebildet sind, um wenigstens bereichsweise am Gaumen anzuliegen. Die Flügel bewirken, dass die Platte in einer vorgegebenen Position in den Gaumen des Säuglings einsetzbar ist. Außerdem fördern die Flügel der vorgeschlagenen Platte einen festen Sitz der Platte im Gaumen des Säuglings, wenn die Platte in den Gaumen eingesetzt ist.

Ferner umfasst die hier vorgeschlagene Platte zumindest zwei Stege, die zumindest abschnittsweise beiderseits der Mittelebene verlaufen, so dass ein zwischen den Stegen verlaufender Freiraum zum Aufnehmen des Vomers ausgebildet ist.

Der hier vorgeschlagenen Platte zum Einsetzen in den Gaumen liegt die Einsicht zugrunde, dass eine Begradigung eines gebogenen Vomers bzw. eine Vermeidung einer Krümmung eines ungebogenen Vomers bei der Behandlung einer Lippen-Kiefer-Gaumen-Segelspalte mit einer Gaumenplatte das Auftreten von Folgeschäden, insbesondere von Atmungsbehinderungen des Patienten im nasalen Bereich entscheidend reduzieren kann.

Die zwei Stege der vorgeschlagenen Platte verlaufen derart zumindest abschnittsweise beiderseits der Mittelebene, dass sie zwischen sich einen Freiraum zum Aufnehmen des Vomers bilden. Beim Einsetzen der Platte in den Gaumen wird das Vomer in den Freiraum aufgenommen und zwischen den zwei Stegen eingefasst. Die Form der Platte ist derart an den Gaumen des Säuglings angepasst, dass sich die Platte insbesondere durch die Flügel am Gaumen abstützt und in mehreren Richtungen am Gaumen fixiert sitzt. Die mehreren Richtungen können beispielsweise mindestens eine Richtung entlang der Mittelebene der Platte und mindestens eine Richtung quer zur Mittelebene umfassen. Der Freiraum definiert eine Verschiebung des Verlaufs des Vomers und ist geeignet ein Vomer aufzunehmen. Die Mittelebene der Platte kann der Sagittalebene des Gaumens entsprechen, wenn die Platte in den Gaumen eingesetzt ist. Durch den festen Sitz der Platte am Gaumen und einen Versatz des Verlaufs der Stege zum gegenwärtigen Verlauf des Vomers kann der Form des Verlaufs des in den Freiraum eingefassten Vomers mittels einer therapeutischen Kraft ein neuer Verlauf aufgezwungen werden. Als therapeutische Kraft wird die Kraft angesehen, die durch die vorgeschlagene Platte auf Bestandteile des Gaumens des Säuglings wie beispielsweise das Vomer oder die Prämaxilla oder beides wirkt, wenn die Platte in den Gaumen des Säuglings eingesetzt ist.

Bei der Behandlung mit der vorgeschlagenen Platte kann eine Veränderung des Verlaufs des Vomers durch eine therapeutische Kraft erreicht werden, indem der Verlauf der Stege der Platte zumindest abschnittsweise von dem Verlauf des Vomers abweicht, wenn die Platte in den Gaumen des Säuglings eingesetzt ist und die Stege das Vomer zumindest bereichsweise verschieben, d. h. die therapeutische Kraft. Eine Verschiebung des Verlaufs des Vomers kann beispielsweise erfolgen, wenn der Freiraum auf einer ersten Seite des Vomers quer zur Mittelebene kleiner ist als der Freiraum auf einer der ersten Seite gegenüberliegenden Seite des Vomers quer zur Mittelebene. In der Folge wird das Vomer beim Einsetzen der Platte in den Gaumen des Säuglings in die Richtung der Seite des Vomers mit dem größeren Freiraum gezwängt, also verschoben. Insbesondere kann eine Verschiebung des Verlaufs des Vomers vorgesehen sein, bei der der Freiraum wenigstens abschnittsweise lediglich auf der zweiten Seite des Vomers quer zur Mittelebene ausgebildet ist. Die Stege können eine dem Vomer angepasste Krümmung aufweisen. Die Krümmung der Stege kann beispielsweise im Vergleich zu der Krümmung des Vomers weniger stark ausgeprägt sein. Besondere Vorteile ergeben sich dadurch, dass die Stege die Krümmung des Vomers durch die therapeutische Kraft reduzieren können. Die Richtung der therapeutischen Kraft kann so durch die Form und Anordnung der Stege gewählt werden, dass eine individuelle Fehlstellung des Vomers gezielt reduziert und zusätzlich oder alternativ korrigiert werden kann. Dadurch werden eine gezielte Veränderung des Verlaufs des Vomers und eine gezielte Begradigung eines gekrümmten Vomers durch die vorgeschlagene Platte ermöglicht.

Die Stege der vorgeschlagenen Platte können entlang der Mittelebene geradlinig und zusätzlich oder alternativ entlang der Mittelebene nebeneinander verlaufend ausgebildet sein. Geradlinig und zusätzlich oder alternativ entlang der Mittelebene nebeneinander verlaufende Stege sind insbesondere von Vorteil, um solange eine therapeutische Kraft auf das Vomer auszuüben, bis der Verlauf des Vomers dem Verlauf des zwischen den Stegen verlaufenden Freiraums entspricht.

Es kann vorgesehen sein, dass ein Anfangszustand des Verlaufs des Vomers und zusätzlich oder alternativ ein gewünschter Endzustand des Verlaufs des Vomers festgelegt werden und der Verlauf des Vomers schrittweise durch eine sukzessive Behandlung mit einer Vielzahl von vorgeschlagenen Platten verändert wird. Der Anfangszustand des Verlaufs des Vomers kann dem anfänglichen Verlauf des Vomers des Säuglings mit Lippen-Kiefer-Gaumen-Segelspalte vor der Behandlung mit der Vielzahl von vorgeschlagenen Platten entsprechen. Ein durch einen Verlauf der Stege festgelegter Freiraum einer ersten Platte der Vielzahl von vorgeschlagenen Platten kann eine erste Verschiebung des Verlaufs des Vomers im Anfangszustand bewirken, wenn die erste Platte in den Gaumen des Säuglings eingesetzt ist. Der Verlauf der Stege der ersten Platte weist einen ersten Versatz gegenüber dem Verlauf des Vomers im Anfangszustand auf. Nachdem der Verlauf der Stege der ersten Platte dem Verlauf des Vomers aufgezwängt wurde, kann die erste Platte der Vielzahl von vorgeschlagenen Platten durch eine zweite Platte der Vielzahl von vorgeschlagenen Platten ausgetauscht werden. Die zweite Platte der Vielzahl von vorgeschlagenen Platten kann durch einen durch einen Verlauf der Stege der zweiten Platte festgelegten Freiraum eine zweite Verschiebung des Verlaufs des Vomers bewirken, wenn die zweite Platte in den Gaumen des Säuglings eingesetzt ist. Der Verlauf der Stege der zweiten Platte weist dabei einen zweiten Versatz gegenüber dem Verlauf des Vomers im Anfangszustand auf, wobei der zweite Versatz größer als der erste Versatz ist. Die zweite Platte kann ebenfalls durch eine weitere Platte mit einer weiteren Verschiebung des Verlaufs des Vomers ausgetauscht werden. Der sukzessive Austausch der Platten kann solange erfolgen bis eine letzte Platte der Vielzahl der vorgeschlagenen Platten eingesetzt wird, wobei die letzte Platte der Vielzahl der vorgeschlagenen Platten einen Verlauf der Stege aufweist, der dem gewünschten Endzustand des Verlaufs des Vomers entspricht, sodass dem Vomer mit der letzten Platte der Verlauf des gewünschten Endzustands aufgezwängt werden kann. Durch eine schrittweise erfolgende Veränderung des Verlaufs des Vomers je Platte können kleinere Verschiebungen des Vomers vorgenommen werden und somit Schmerzen für den Säugling durch die Behandlung mit einer jeweiligen Platte reduziert werden. Außerdem ist die mögliche Verschiebung des Verlaufs des Vomers durch lediglich eine Platte auf eine maximale Verschiebung begrenzt. Durch eine sukzessive Behandlung mit einer Vielzahl von Platten kann die maximale Verschiebung einer einzelnen Platte überwunden werden und eine größere Gesamtverschiebung erreicht werden.

Darüber hinaus kann der Verlauf des Vomers durch einen an den Verlauf des Vomers angepassten Verlauf der Stege beibehalten werden. Dies ist beispielsweise sinnvoll, wenn der Verlauf des Vomers bereits in der gewünschten Form vorliegt und in dieser Form gehalten werden soll.

Die beiderseits der senkrecht zur Platte gedachten Mittelebene verlaufenden Flügel der vorgeschlagenen Platte, die derart ausgebildet sind, um wenigstens bereichsweise am Gaumen anzuliegen, können insbesondere zum Fixieren der Platte im Gaumen des Säuglings eingerichtet sein. Die Flügel können aufgrund ihrer Form mit ihren Oberseiten wenigstens bereichsweise an den Gaumen des Säuglings anschmiegbar sein. Vorzugsweise können die Flügel derart ausgebildet sein, dass sie beim Einsetzen der Platte in den Gaumen mit jeweils einem lateralen Oberkiefersegment des Säuglings kontaktiert werden. Das heißt, die Oberseiten der Flügel können eingerichtet sein, zum Oberkiefer hin, d.h. in kranial, mit den zum Unterkiefer hin, d.h. in kaudal, gerichteten Flächen der lateralen Oberkiefersegmente zu kontaktieren. Ferner können die Oberseiten der Flügel und zusätzlich oder alternativ die Form der Flügel an die Oberfläche der Oberkiefersegmente passend, vorzugsweise passgenau, ausgebildet sein. Dies hat den Vorteil, dass der Halt der Platte am Oberkiefer des Säuglings beim Einsetzen der Platte unterstützt werden kann und dass die passende Form der Flügel den Tragekomfort des Säuglings verbessert.

Typischerweise kann die Platte einstückig ausgebildet sein und zusätzlich oder alternativ aus Kunstoff bestehen. Beispiele sind Polyetheretherketon (PEEK)und/oder Kunstharz.
In einer Ausführungsform kann die Platte eine Aufnahme zum Aufnehmen der Prämaxilla aufweisen, wobei die Aufnahme wenigstens eine muldenartige Vertiefung umfasst. Die Aufnahme kann vorzugsweise zum Aufnehmen der Prämaxilla des Säuglings und zum Verringern einer Fehlstellung der Prämaxilla des Säuglings dienen. Die muldenartige Vertiefung ist dabei derart ausgebildet, dass die Prämaxilla des Säuglings in der muldenartigen Vertiefung angeordnet ist, wenn die Platte in den Gaumen des Säuglings eingesetzt ist.

Es kann vorgesehen sein, dass die Form und Anordnung der muldenartigen Vertiefung derart gewählt wird, dass die muldenartige Vertiefung einen Versatz gegenüber der Mittelebene der Platte aufweist, sodass durch die muldenartige Vertiefung eine therapeutische Kraft auf die Prämaxilla des Säuglings ausgeübt werden kann, wenn die Platte in den Gaumen des Säuglings eingesetzt ist. Die Form und Anordnung der muldenartigen Vertiefung können so gewählt werden, dass durch die therapeutischen Kraft eine individuelle Fehlstellung der Prämaxilla gezielt reduziert und zusätzlich oder alternativ korrigiert werden kann.

Die muldenartige Vertiefung kann derart ausgeprägt sein, dass die Form und Anordnung der der muldenartigen Vertiefung der Form und Anordnung der Prämaxilla entspricht. Dies ist beispielsweise sinnvoll, wenn die gegenwärtige Form und Anordnung der Prämaxilla bereits der gewünschten Form und Anordnung entspricht und in dieser Form und Anordnung gehalten werden soll.

Je nach Form und Anordnung der muldenartigen Vertiefung und dem Verlauf der Stege können mit der vorgeschlagenen Platte verschiedene Fehlstellungen des Vomers und der Prämaxilla gezielt behandelt werden. Durch eine vorgeschlagene Platte mit beispielsweise einem Versatz der muldenartigen Vertiefung zur Mittelebene der Platte und einem Versatz des Verlaufs der Stege zur Mittelebene der Platte können gleichzeitig der Verlauf des Vomers sowie die Anordnung der Prämaxilla verändert werden, wenn die Platte in den Gaumen des Säuglings eingesetzt ist. Ebenso kann es vorgesehen sein, dass die vorgeschlagene Platte lediglich einen Versatz des Verlaufs der Stege zur Mittelebene aufweist, wodurch lediglich eine Verschiebung des Verlaufs des Vomers bewirkt wird. Hierbei kann die muldenartige Vertiefung ohne Versatz zur Mittelebene angeordnet sein oder der Form und Anordnung der Prämaxilla entsprechen. Dadurch kann eine Verschiebung der Prämaxilla durch die muldenartige Vertiefung verhindert werden, während der Verlauf des Vomers verschoben werden kann, wenn die Platte in den Gaumen des Säuglings eingesetzt ist. In analoger Weise kann die Platte einen Verlauf der Stege ohne Versatz zur Mittelebene oder einen Verlauf der Stege, der dem Verlauf des Vomers entspricht, und einen Versatz der muldenartigen Vertiefung zur Mittelebene der Platte aufweisen. Somit ist es möglich, den Verlauf des Vomers beizubehalten und die Form und Anordnung der Prämaxilla zu verändern. Dies ermöglicht eine Behandlung von einer Vielzahl von Fehlstellungen im Oberkiefer des Säuglings mit der vorgeschlagenen Platte.

Darüber hinaus kann die Aufnahme an einem in eine Richtung entlang der Mittelebene verlaufenden Ende einer der Stege oder beider Stege angeordnet sein. Der eine Steg kann an diesem Ende abgerundet sein. Vorzugsweise kann das Ende des Steges derart ausgebildet sein, dass es an die jeweilige anatomische Gegebenheit des Gaumens wie beispielsweise die Nasenmuschel angepasst ist. Es können aber auch beide Stege an diesen Enden abgerundet sein. Vorzugsweise können beide Stege an diesen Enden derart ausgebildet sein, dass das jeweilige Ende an die jeweilige anatomische Gegebenheit des Gaumens wie beispielsweise die Nasenmuschel angepasst ist. Die in die Richtung entlang der Mittelebene verlaufenden Enden der Stege können unmittelbar in die Aufnahme übergehen oder einen Abstand zur Aufnahme aufweisen. Vorzugsweise kann der Übergang zwischen den entlang der Mittelebene verlaufenden Enden der Stege und der Aufnahme derart ausgebildet sein, dass der Übergang an die jeweilige anatomische Gegebenheit des Gaumens wie beispielsweise die Nasenmuschel angepasst ist. Derartige Anpassungen des Steges bzw. des Übergangs der Stege zu der Aufnahme können das Anschmiegen der Platte an den Gaumen des Säuglings verbessern und Schmerzen des Säuglings verringern, wenn die Platte in den Gaumen des Säuglings eingesetzt wird oder ist..

Die muldenartige Vertiefung kann derart ausgebildet sein, dass eine Innenfläche der muldenartigen Vertiefung die Prämaxilla umlaufend umschließt, wenn die Platte in den Gaumen eingesetzt ist. Typischerweise kann das entlang der Mittelebene verlaufende Ende einer der Stege in die Körperrichtung nach vorn, d.h. in anterior, zeigen, wenn die Platte in den Gaumen eingesetzt ist. Eine derartige Anordnung der Aufnahme in der Platte bietet den Vorteil, dass sich die Aufnahme und damit die muldenartige Vertiefung auf der Höhe der Prämaxilla angeordnet sein kann, wenn die Platte in den Gaumen eingesetzt ist. Ferner ermöglicht ein umlaufendes Umschließen der Prämaxilla durch die muldenartige Vertiefung er einen besonders vorteilhaften Halt der Prämaxilla, wenn die Platte in den Gaumen des Säuglings eingesetzt ist. Zudem kann ein Herausgleiten der Prämaxilla aus der muldenartigen Vertiefung verhindert werden.

Darüber hinaus kann es vorgesehen sein, dass eine quer zur Mittelebene bestimmte maximale Breite eines die Aufnahme enthaltenden Aufnahmebereichs der Platte höchstens 80 Prozent, vorzugsweise höchstens 70 Prozent, besonders bevorzugt höchstens 60 Prozent einer quer zur Mittelebene bestimmten maximalen Breite der Platte beträgt. Die maximale Breite der Platte kann beispielsweise höchstens 8 cm, vorzugsweise höchstens 6 cm, besonders bevorzugt höchstens 5 cm betragen. Eine verringerte Breite der Platte im Bereich des Aufnahmebereichs ist insofern vorteilhaft, als dass die Stellung der Prämaxilla unabhängig von den vorderen Bereichen der lateralen Kiefersegmente durch die Platte veränderbar ist, wenn die Platte in den Gaumen eingesetzt ist. Es kann aber durchaus auch vorgesehen sein, dass die Platten derart ausgebildet sind, dass die lateralen Segmente unabhängig von der Prämaxilla bewegt werden können, wenn die Platte in den Gaumen eingesetzt ist. Beispielsweise können Verlagerungen der lateralen Segmente mittels einer digitalen oder analogen Modell-Operation unabhängig von dem Vomer simuliert werden. In der Modell-Operation können die lateralen Segmente individuell verlagert werden, insbesondere derart, dass eine Stellung der lateralen Segmente zueinander zur optimalen Einbringung der Prämaxilla einstellbar ist. Durch eine individuelle Einstellbarkeit der lateralen Segmente können Fehlstellungen im Gaumen des Säuglings besser und vor allem gezielter behoben oder zumindest reduziert werden.

In einer weiteren Ausführungsform können die Flügel eine Kontaktfläche der Platte mit dem Gaumen aufweisen. Die Stege können an einer Seite der Platte abstehen, so dass sie über die Kontaktfläche erhaben sind. Die Kontaktfläche kann sich typischerweise über die Oberseite der Flügel erstrecken. Vorzugsweise können die Stege in kranial von der Kontaktfläche abstehen. Die Stege können beispielsweise stufenartig in kranial von der Kontaktfläche abstehen. Durch die Kontaktfläche kann verhindert werden, dass sich die Stege zu weit in kranial in den nasalen Bereich des Säuglings erstrecken. Denn zu weit in kranial reichende Stege können unter Umständen die nasalen Atemwege des Säuglings versperren und eine erhebliche Lebensgefahr für den Säugling durch Erstickung hervorrufen.

In einer weiteren Ausführungsform kann eine senkrecht zur Kontaktfläche und ausgehend von der Kontaktfläche bestimmte Höhe der Stege mindestens 0,4 cm, vorzugsweise mindestens 0,8 cm, besonders bevorzugt mindestens 1,2 cm und zusätzlich oder alternativ höchstens 2 cm, vorzugsweise höchstens 1,8 cm, besonders bevorzugt höchstens 1,5 cm betragen. Alternativ kann sich die Höhe der Stege entlang der Mittelebene um höchstens 50 Prozent, vorzugsweise um höchstens 30 Prozent, besonders bevorzugt um höchstens 10 Prozent einer minimalen oder einer maximalen Höhe der Stege ändern. Eine maximale Höhe eines ersten Steges kann beispielsweise um höchstens 50 Prozent, vorzugsweise um höchstens 30 Prozent, besonders bevorzugt um höchstens 10 Prozent von einer maximalen Höhe eines zweiten Steges abweichen. Alternativ oder zusätzlich kann die Höhe der Stege wenigstens das Dreifache, vorzugsweise wenigstens das Zweifache, besonders bevorzugt wenigstens das Einfache einer quer zur Mittelebene bestimmten minimalen Breite der Stege betragen. Stege mit einer zu geringen Höhe können beispielsweise dazu führen, dass das Vomer nicht im ausreichenden Maße lateral gestützt werden kann, sodass eine Krümmung bzw. eine Begradigung des Vomers nicht verhindert werden bzw. erfolgen kann. Stege, die eine maximale Höhe nicht überschreiten, können ein Versperren der nasalen Atemwege und eine erhebliche Lebensgefahr für den Säugling durch Erstickung vermeiden, wenn die die Platte in den Gaumen eingesetzt ist.

In einer weiteren Ausführungsform können die Stege quer zur Mittelebene voneinander beabstandet sein, wobei ein minimaler Abstand der Stege voneinander mindestens 0,3 cm, vorzugsweise mindestens 0,5 cm, besonders bevorzugt mindestens 0,7 cm und/oder höchstens 2 cm, vorzugsweise höchstens 1,5 cm, besonders bevorzugt höchstens 1 cm betragen kann. Alternativ kann sich der Abstand der Stege voneinander entlang der Mittelebene um weniger als das Zweifache, vorzugsweise um weniger als das Einfache, besonders bevorzugt um weniger als die Hälfte des minimalen Abstands der Stege ändern. Typischerweise kann ein minimaler Abstand der Stege zueinander vorgesehen sein, damit das Vomer in den zwischen den Stegen verlaufenden Freiraum passt, wenn die Platte in den Gaumen eingesetzt ist.

Die Stege können alternativ jeweils eine entlang der Mittelebene bestimmte Länge und eine quer zur Mittelebene bestimmte Breite haben, wobei die Länge eines jeden Stegs wenigstens das Zweifache, vorzugsweise wenigstens das Dreifache, besonders bevorzugt wenigstens das Vierfache seiner Breite betragen kann. Ferner kann es vorgesehen sein, dass sich die Breite eines jeden Stegs jeweils um weniger als das Zweifache, vorzugsweise um weniger als das Einfache, besonders bevorzugt um weniger als die Hälfte einer minimalen Breite der Stege ändert.

In einer weiteren Ausführungsform können die Flügel an von der Mittelebene abgewandten Seiten der Stege angeordnet sein. Die Flügel können beispielsweise derart ausgebildet sein, dass sie an die Stege heranreichen. Flügel, die an die Stege heranreichen bieten beispielsweise den Vorteil, dass die Platte ein hohes Maß an Stabilität aufweist. Dadurch kann die Platte Kräften standhalten, die beim Einsetzen der Platte wirken, ohne dabei zu zerbrechen.

Alternativ kann eine entlang der Mittelebene bestimmte maximale Länge der Flügel höchstens 100 Prozent, vorzugsweise höchstens 90 Prozent, besonders bevorzugt höchstens 80 Prozent einer entlang der Mittelebene bestimmten Länge einer der Stege oder alternativ aller Stege betragen. Durch eine Begrenzung der Länge der Flügel können die lateralen Kiefersegmente wenigstens bereichsweise von der Platte ausgespart werden. Dies ermöglicht, dass die Prämaxilla unabhängig von den lateralen Kiefersegmenten oder die lateralen Kiefersegmente unabhängig von der Prämaxilla durch die Verwendung der Platte verschoben werden können.

Die maximale Länge der Flügel kann einen entlang der Mittelebene ausgerichteten Längenabschnitt definieren. In dem Längenabschnitt kann sich eine quer zur Mittelebene bestimmte maximale Breite der Platte um mindestens 40 Prozent, vorzugsweise um mindestens 50 Prozent, besonders bevorzugt um mindestens 60 Prozent reduzieren, wobei eine entlang der Mittelebene bestimmte Länge des Längenabschnitts höchstens 25 Prozent, vorzugsweise höchstens 15 Prozent, besonders bevorzugt höchstens zehn Prozent einer entlang der Mittelebene bestimmten maximalen Länge der Platte betragen kann.

In einer weiteren Ausführungsform kann die Platte eine Halteeinrichtung zum Einspannen einer Spannvorrichtung zur Krafteinwirkung auf die Platte entlang der Mittelebene umfassen, wobei die Halteeinrichtung an einem von den Stegen abgewandten und in eine Richtung entlang der Mittelebene verlaufenden Ende der Aufnahme angeordnet sein kann. Die Halteeinrichtung kann beispielsweise anterior von der Aufnahme abstehen, wenn die Platte in den Gaumen eingesetzt ist. Die Spannvorrichtung kann an der Halteeinrichtung mit der Platte gekoppelt sein. Über eine Zugspannung der Spannvorrichtung kann eine Kraft über die Halteeinrichtung auf die Platte übertragbar sein. Üblicherweise kann die Zugspannung bzw. der Kraftübertrag in posterior ausgeübt werden, wenn die Platte in den Gaumen eingesetzt ist. Es können aber auch Krafteinwirkungen in anterior vorgesehen sein. Zusätzlich kann aber auch eine myofunktionelle Krafteinwirkung der Zunge des Säuglings auf die Platte berücksichtigt und ausgenutzt werden, wenn die Platte in den Gaumen des Säuglings eingelegt ist. Beispiele für eine Spannvorrichtung können Gummibänder oder Federn sein. Die Kraft kann beispielsweise als therapeutische Kraft auf das Vomer und zusätzlich oder alternativ auf die Prämaxilla übertragen werden. Dies ermöglicht, dass das Vomer und zusätzlich oder alternativ die Prämaxilla entlang der Mittelebene durch die Verwendung der Platte verschoben werden können. Dadurch können auch auftretende Fehlstellungen der Prämaxilla und des Vomers in anterior oder in posterior korrigiert und behoben werden. Die korrigierende Wirkung der eingesetzten Platte kann hierbei durch die myofunktionelle Krafteinwirkung der Zunge des Säuglings auf die Platte begünstigt werden. Außerdem kann eine therapeutische Kraft auf eines der oder beide lateralen Kiefersegmente ausgeübt werden, um die Position des oder der lateralen Kiefersegmente zu korrigieren.

Die Halteeinrichtung kann beispielsweise zwei Fixiervorsprünge aufweisen, die beim Einsetzen der Platte in den Gaumen anterior vorstehen. Die Fixiervorsprünge können beispielsweise beiderseits der Mittelebene angeordnet sein. Die Anordnung der Fixiervorsprünge kann die Richtung der therapeutischen Kraft quer zur Mittelebene beeinflussen. Dies ermöglicht eine weitere Einstellbarkeit der therapeutischen Kraft, wodurch beispielsweise eine Verschiebung der lateralen Kiefersegmente individueller gestaltet werden kann, wenn die Platte in den Gaumen eingesetzt ist.

Ferner wird ein Satz von Platten zum sequenziellen Begradigen des Vomers eines Säuglings mit Lippen-Kiefer-Gaumen-Segelspalte vorgeschlagen. Der Satz von Platten kann dabei helfen, Fehlstellungen des Vomers sukzessive zu behandeln. Die Verläufe der Stege der Platten des Satzes von Platten können unterschiedliche Versätze gegenüber einem Verlauf der Stege einer ersten Platte des Satzes von Platten aufweisen. Der jeweilige Versatz des Verlaufs der Stege gegenüber dem Verlauf der Stege der ersten Platte kann mit jeder weiteren Platte zunehmen, sodass eine therapeutische Kraft einer jeden nachfolgenden Platte den Verlauf des Vomers schrittweise verändert.

Der Satz von Platten umfasst mindestens eine erste zuvor beschriebene Platte und eine zweite zuvor beschriebene Platte, wobei sich ein erster räumlicher Verlauf der Stege der ersten Platte in eine Richtung entlang der Mittelebene der ersten Platte von einem zweiten Verlauf der Stege der zweiten Platte in eine Richtung entlang der Mittelebene der zweiten Platte voneinander unterscheidet. Durch unterschiedliche Verläufe der Stege der Platten des Satzes von Platten können unterschiedliche therapeutische Kräfte bewirkt werden, wenn die jeweilige Platte in den Gaumen eingesetzt wird. Dadurch wird ermöglicht, dass das Vomer durch Verwendung der ersten Platte und nachfolgendem Austausch der ersten Platten durch die zweite Platte sequenziell begradigt werden kann. Es ist ersichtlich, dass noch weitere Platten zum sequenziellen Begradigen des Vomers mit unterschiedlichen Verläufen der Stege der jeweiligen Platte zur Verringerung einer Fehlstellung des Vomers vorgesehen sein können. Bei der sequenziellen Verwendung eines Satz von Platten mit mehreren Platten kann es vorgesehen sein, dass sich ein Abstand zwischen den Stegen und damit der Freiraum zum Aufnehmen des Vomers der jeweiligen Platte mit jeder nachfolgenden Platte vergrößert. Dies bietet den Vorteil, dass sich das durch Wachstum des Säuglings vergrößernde Volumen des Vomers des Säuglings mit jeder nachfolgenden Platte des Satzes von Platten berücksichtigt werden kann. Darüber hinaus können ebenfalls Fehlstellungen der Prämaxilla und der lateralen Kiefersegmente durch den Satz von Platten behoben oder zumindest verringert werden.

Alternativ können die Stege der ersten Platte einen ersten und einen zweiten Steg umfassen. Die Stege der zweiten Platte können einen ersten und einen zweiten Steg umfassen. Jeder der Stege kann jeweils ein in eine Richtung entlang der Mittelebene verlaufendes erstes Ende und ein dem ersten Ende gegenüberliegendes zweites Ende aufweisen. Das erste Ende des ersten Stegs der ersten Platte kann gegenüber dem zweiten Ende des ersten Stegs der ersten Platte quer zur Mittelebene der ersten Platte um einen ersten Querversatz versetzt angeordnet sein. Das erste Ende des zweiten Stegs der ersten Platte kann gegenüber dem zweiten Ende des zweiten Stegs der ersten Platte quer zur Mittelebene der ersten Platte um einen zweiten Querversatz versetzt angeordnet sein. Das erste Ende des ersten Stegs der zweiten Platte kann gegenüber dem zweiten Ende des ersten Stegs der zweiten Platte quer zur Mittelebene der zweiten Platte um einen dritten Querversatz versetzt angeordnet sein. Das erste Ende des zweiten Stegs der zweiten Platte kann gegenüber dem zweiten Ende des zweiten Stegs der zweiten Platte quer zur Mittelebene der zweiten Platte um einen vierten Querversatz versetzt angeordnet sein. Der erste Querversatz kann um mindestens 30 Prozent, vorzugsweise um mindestens zehn Prozent, besonders bevorzugt um mindestens fünf Prozent kleiner sein als der dritte Querversatz. Zusätzlich oder alternativ kann der zweite Querversatz um mindestens 20 Prozent, vorzugsweise um mindestens zehn Prozent, besonders bevorzugt um mindestens fünf Prozent kleiner sein als der vierte Querversatz. Zusätzlich oder alternativ kann das erste Ende des ersten Stegs der ersten Platte gegenüber dem zweiten Ende des ersten Stegs der ersten Platte entlang einer Richtung parallel zur Mittelebene der ersten Platte um einen ersten Längsversatz versetzt angeordnet sein. Das erste Ende des zweiten Stegs der ersten Platte kann gegenüber dem zweiten Ende des zweiten Stegs der ersten Platte entlang einer Richtung parallel zur der Mittelebene der ersten Platte um einen zweiten Längsversatz versetzt angeordnet sein. Das erste Ende des ersten Stegs der zweiten Platte kann gegenüber dem zweiten Ende des ersten Stegs der zweiten Platte entlang einer Richtung parallel zur der Mittelebene der zweiten Platte um einen dritten Längsversatz versetzt angeordnet sein. Das erste Ende des zweiten Stegs der zweiten Platte kann gegenüber dem zweiten Ende des zweiten Stegs der zweiten Platte entlang einer Richtung parallel zur der Mittelebene der zweiten Platte um einen vierten Längsversatz versetzt angeordnet sein. Der erste Längsversatz kann um mindestens 15 Prozent, vorzugsweise um mindestens zehn Prozent, besonders bevorzugt um mindestens fünf Prozent kleiner sein als der dritte Längsversatz. Zusätzlich oder alternativ kann der zweite Längsversatz um mindestens 15 Prozent, vorzugsweise um mindestens zehn Prozent, besonders bevorzugt um mindestens fünf Prozent kleiner sein als der vierte Längsversatz.

Der Längsversatz bzw. der Querversatz ermöglicht eine gezielte Verringerung der Fehlstellung der Prämaxilla. Typischerweise ist es dadurch möglich, die Prämaxilla entlang der Mittelebene und zusätzlich oder alternativ entlang der orthogonal auf der Mittelebene liegenden Ebene zu verschieben. Üblicherweise kann die gezielte Verringerung pro Platte höchstens 0,3 cm, vorzugsweise höchstens 0,2 cm, besonders bevorzugt höchstens 0,1 cm und zusätzlich oder alternativ mindestens 0,05 cm betragen.

Ferner wird ein Verfahren zum Herstellen einer Platte zum Einsetzen in den Gaumen eines Säuglings mit Lippen-Kiefer-Gaumen-Segelspalte zum Begradigen des Vomers des Säuglings vorgeschlagen, welches folgende Schritte umfasst:
Herstellen und Ausmessen eines Abdrucks des Oberkiefers des Säuglings zum Bestimmen einer räumlichen Anordnung des Vomers, der Prämaxilla und der beiden lateralen Oberkiefersegmente des Säuglings relativ zueinander,
Bestimmen einer quer zur Sagittalebene des Säuglings bestimmten Vomerquerfehlstellung des zur Prämaxilla gerichteten Endes des Vomers und zusätzlich oder alternativ
Bestimmen einer entlang der Sagittalebene des Säuglings bestimmten Vomerlängsfehlstellung des zur Prämaxilla gerichteten Endes des Vomers, und
Herstellen einer zuvor beschriebenen Platte,
wobei die Stege der Platte als ein erster Steg und als ein zweiter Steg mit jeweils einem in eine Richtung entlang der Mittelebene verlaufenden ersten Ende und einem dem ersten Ende gegenüberliegenden zweiten Ende derart ausgebildet werden, dass
(i) das erste Ende des ersten Stegs gegenüber dem zweiten Ende des ersten Stegs um einen quer zur Mittelebene der Platte bestimmten ersten Querversatz versetzt angeordnet ist und dass das erste Ende des zweiten Stegs gegenüber dem zweiten Ende des zweiten Stegs um einen zur Mittelebene der Platte bestimmten zweiten Querversatz versetzt angeordnet ist, wobei der erste Querversatz und der zweite Querversatz jeweils abhängig von der zuvor bestimmten Vomerquerfehlstellung gewählt wird; und zusätzlich oder alternativ
(ii) das erste Ende des ersten Stegs gegenüber dem zweiten Ende des ersten Stegs um einen entlang einer Richtung parallel zur Mittelebene der Platte bestimmten ersten Längsversatz versetzt angeordnet ist und dass das erste Ende des zweiten Stegs gegenüber dem zweiten Ende des zweiten Stegs um einen entlang einer Richtung parallel zur Mittelebene der Platte bestimmten zweiten Längsversatz versetzt angeordnet ist,
wobei der erste Längsversatz und der zweite Längsversatz jeweils abhängig von der zuvor bestimmten Vomerlängsfehlstellung gewählt wird.

Typischerweise wird bei dem Verfahren zum Herstellen vor der Herstellung der Platte zunächst ein Abdruck des Oberkiefers des Säuglings hergestellt. Der Abdruck kann beispielsweise digital und zusätzlich oder alternativ analog genommen werden. Vorzugsweise kann der analoge Abdruck digitalisiert werden. Aus dem Abdruck können die Fehlstellung des Vomers, der Prämaxilla und der lateralen Kiefersegmente ausgemessen werden. Je nach Ausmaß der jeweiligen Fehlstellung kann eine unterschiedliche Anzahl von Platten zur Verringerung der jeweiligen Fehlstellung geplant und hergestellt werden.

Zusätzlich kann das Verfahren folgende Schritte umfassen:
Herstellen einer ersten Platte nach dem zuvor beschriebenen Verfahren und einer zweiten Platten nach dem zuvor beschriebenen Verfahren,
wobei der erste Querversatz des ersten Endes des ersten Stegs der ersten Platte kleiner gewählt werden kann als der erste Querversatz des ersten Endes des ersten Stegs der zweiten Platte und wobei der zweite Querversatz des ersten Endes des zweiten Stegs der ersten Platte kleiner gewählt werden kann als der zweite Querversatz des ersten Endes des zweiten Stegs der zweiten Platte und zusätzlich oder alternativ
wobei der erste Längsversatz des ersten Endes des ersten Stegs der ersten Platte kleiner gewählt werden kann als der erste Längsversatz des ersten Endes des ersten Stegs der zweiten Platte und wobei der zweite Längsversatz des ersten Endes des zweiten Stegs der ersten Platte kleiner gewählt werden kann als der zweite Längsversatz des ersten Endes des zweiten Stegs der zweiten Platte.

Ein Ausführungsbeispiel eines Satzes von Platten der hier vorgeschlagenen Art und des hier vorgeschlagenen Verfahrens sind in den Figuren dargestellt und werden anhand der nachfolgenden Beschreibung näher erläutert. Es zeigen:
- Fig. 1: einen Abdruck des Oberkiefers eines Säuglings mit einer Fehlstellung des Vomers und der Prämaxilla in der Schrägansicht;
- Fig. 2A: eine perspektivische Darstellung eines Ausführungsbeispiels einer ersten Platte des vorgeschlagenen Satzes von Platten in der Vorderansicht;
- Fig. 2B: eine perspektivische Darstellung einer zweiten Platte des vorgeschlagenen Satzes von Platten in der Vorderansicht;
- Fig. 2C: eine perspektivische Darstellung der ersten Platte mit einer Überlagerung der zweiten Platte des vorgeschlagenen Satzes von Platten in der Vorderansicht;
- Fig. 3A: eine perspektivische Darstellung der ersten Platte des vorgeschlagenen Satzes von Platten in der Hinteransicht;
- Fig. 3B: eine perspektivische Darstellung der zweiten Platte des vorgeschlagenen Satzes von Platten in der Hinteransicht;
- Fig. 4A-D: schematische Darstellungen von Anordnungen der Aufnahme sowie der zwei Stege einer Platte des vorgeschlagenen Satzes von Platten in der Draufsicht; sowie
- Fig. 5A-D: Längsquerschnitte einer Platte des vorgeschlagenen Satzes von Platten mit verschiedenen Übergängen zwischen den Stegen und der Aufnahme.

Anhand der Figuren wird ein Ausführungsbeispiel eines Satzes von Platten zum sequenziellen Begradigen des Vomers eines Säuglings mit Lippen-Kiefer-Gaumen-Segelspalte und zum Verringern einer Fehlstellung der Prämaxilla des Säuglings gezeigt. Durch die sequenzielle Verwendung des Satzes von Platten können das Vomer des Säuglings begradigt und eine Krümmung des Vomers des Säuglings verhindert sowie die Fehlstellung der Prämaxilla des Säuglings reduziert werden.

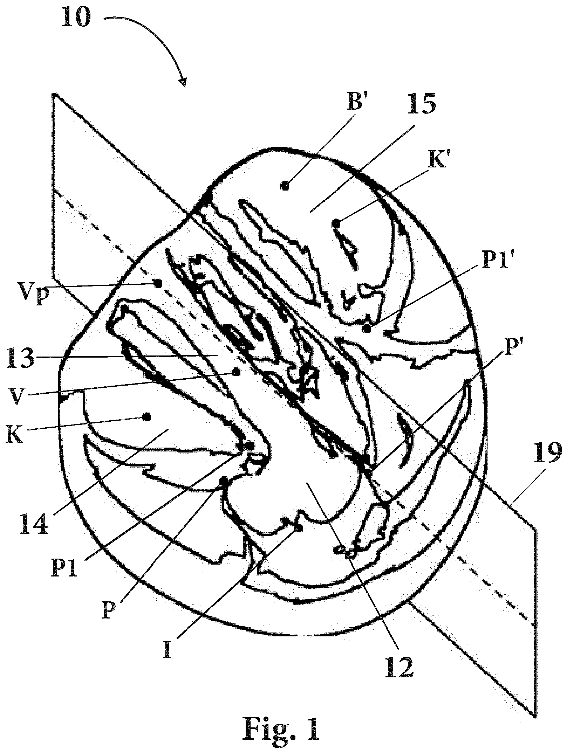

In Figur 1 ist ein digitaler Abdruck 10 eines Oberkiefers eines Säuglings mit Lippen-Kiefer-Gaumen-Segelspalte in der Schrägansicht gezeigt. Der Abdruck 10 weist eine kreisförmige Außenkontur auf und definiert eine Abdruckmittelebene 19 entlang seines Durchmessers. Die Abdruckmittelebene 19 entspricht der Sagittalebene des Säuglings. Beiderseits und entlang der Abdruckmittelebene 19 verlaufend sind zwei erhabene laterale Oberkiefersegmente 14, 15 abgebildet, die in jeweils einem sich gegenüberliegenden äußeren Bereich des Abdrucks 10 ausgebildet sind. Zwischen den Oberkiefersegmenten 14, 15 ist das Vomer 13 eines Säuglings als eine stegartige Erhebung abgebildet. Ein erstes Ende des abgebildeten Vomers 13 ist mittig zwischen den Oberkiefersegmenten 14, 15 angeordnet. Ausgehend von dem ersten Ende erstreckt sich das Vomer 13 entlang der Abdruckmittelebene 19 mit einem Winkel zur Abdruckmittelebene 19 von ca. 10 Grad. An einem dem ersten Ende gegenüberliegenden zweiten Ende des Vomers 13 ist die Prämaxilla 12 des Säuglings erhaben dargestellt. Die Prämaxilla 12 ist mit dem zweiten Ende des Vomers 13 verbunden und umfasst zwei kugelartige Ausformungen. Die kugelartigen Ausformungen sind auf einer von dem zweiten Ende des Vomers 13 abgewandten Seite der Prämaxilla 12 angeordnet sind. Ferner erstreckt sich die Prämaxilla 12 in eine Richtung mit einem Winkel zur Abdruckmittelebene 19 von ca. 30 Grad. Die Prämaxilla 12 und das zweite Ende des Vomers 13 umgeben zumindest bereichsweise ein erstes 14 der zwei lateralen Oberkiefersegmente 14,15 in einem kurzen Abstand. Ein derart kurzer Abstand begünstigt typischerweise ein zügiges Zusammenwachsen der Prämaxilla 12, des Vomers 13 und des ersten Oberkiefersegments 14. Ein aus dieser Fehlstellung der Prämaxilla 12 und des Vomers 13 resultierender großer Abstand zu einem dem ersten Oberkieferelement 14 gegenüberliegenden zweiten Oberkieferelements 15 verzögert jedoch ein Zusammenwachsen der Prämaxilla 12, des Vomers 13 und des zweiten Oberkiefersegments 15. Aus dieser Fehlstellung resultiert meist eine Lippen-Kiefer-Gaumen-Segelspalte im Oberkiefer des Säuglings. Eine derartige Fehlstellung kann mit dem vorgeschlagenen Satz von Platten behandelt werden.

Durch einen wie in Figur 1 dargestellten Abdruck 10 des Oberkiefers eines Säuglings mit Lippen-Kiefer-Gaumen-Segelspalte können die räumliche Anordnung und das Ausmaß der Fehlstellung der Prämaxilla 12, des Vomers 13 und der lateralen Kiefersegmente 14, 15 ausgemessen werden. Für die Ausmessungen können die in der Figur 1 dargestellten Punkte herangezogen werden. Diese Punkte beschreiben einen Basispunkt B', einen posterioren Punkt Vp bzw. einen Mittelpunkt V des Vomers 13, einen Gingivalsulcuspunkt K bzw. K' des ersten bzw. zweiten Oberkieferelements 14, 15, Alveolarspaltpolpunkte P1 bzw. P1', anteriore Punkte des Alverolarkamms P bzw. P' und einen Interinzialpunkt I der Prämaxilla 12. Im Anschluss kann eine quer zur Abdruckmittelebene 19 bestimmte Vomerquerfehlstellung und eine entlang der Abdruckmittelebene 19 bestimmte Vomerlängsfehlstellung des zur Prämaxilla 12 gerichteten Endes des Vomers 13 bestimmt werden. Ferner kann aus dem Abdruck 10 eine Fehlstellung der Prämaxilla 12 und der lateralen Kiefersegmente 14, 15 bestimmt werden. Die Vomerquerfehlstellung und/oder die Fehlstellung der Prämaxilla 12 und der lateralen Kiefersegmente 14, 15 können beispielsweise durch die in Tabelle 1 illustrierten Abstände der verschiedenen Punkte quer zur Abdruckmittelebene 19 beschrieben und bestimmt werden. Der in Tabelle 1 und 2 beschriebene erste Abdruck wurde von dem Gaumen des Säuglings genommen. Die übrigen Abdrücke sind durch Modell-Operationen entworfen. Allerdings kann es auch vorgesehen sein, dass die einzelnen Abdrücke jeweils von dem Gaumen des Säuglings genommen werden. Die Vomerlängsfehlstellung kann beispielsweise durch die in Tabelle 2 aufgeführten Abstände des Basispunkts B' zum posterioren Punkt Vp des Vomers 13 entlang der Abdruckmittelebene 19 ermittelt werden. Beispielhaft sind die entsprechenden Abstände für sieben digitale Abdrücke dargestellt. Die angegebenen Werte sind auf die Nachkommastelle gerundet. Abhängig vom Ausmaß der jeweiligen Fehlstellung kann eine Anzahl von Platten der vorgeschlagenen Art geplant und festgelegt werden, um einen Satz von Platten zum sequenziellen Begradigen der Prämaxilla 12 und des Vomers 13 des Säuglings mit Lippen-Kiefer-Gaumen-Segelspalte und zum sequenziellen Verringern der Fehlstellung der Prämaxilla 12, des Vomers 13 und der lateralen Kiefersegmente 14, 15 des Säuglings herzustellen.

**Tabelle 1: Abstände verschiedener Punkte der Abdrücke quer zur Abdruckmittelebene**

| | Abstand Vp-I quer zur Mittelebene in cm | Abstand V-K' quer zur Mittelebene in cm | Abstand V-K' quer zur Mittelebene in cm | Abstand P1-P1' quer zur Mittelebene in cm | Abstand P-P' quer zur Mittelebene in cm |
|---|---|---|---|---|---|
| Erster Abdruck | 1,4 | 0,9 | 2 | 1,9 | 0 |
| Zweiter Abdruck | 1 | 1,1 | 1,7 | 1,3 | 0,2 |
| Dritter Abdruck | 0,4 | 1,3 | 1,5 | 1 | 0,6 |
| Vierter Abdruck | 0,3 | 1,3 | 1,5 | 0,6 | 0,5 |
| Fünfter Abdruck | 0,3 | 1,3 | 1,5 | 0,5 | 0,3 |
| Sechster Abdruck | 0,2 | 1,4 | 1,4 | 0,3 | 0,2 |
| Siebter Abdruck | 0,3 | 1,4 | 1,4 | 0,2 | 0 |

**Tabelle 2: Abstände des posterioren Punkts Vp zum Basispunkt B' entlang der Abdruckmittelebene**

| | Abstand B'-Vp entlang der Mittelebene in cm |
|---|---|
| Erster Abdruck | 0,5 |
| Zweiter Abdruck | 0,5 |
| Dritter Abdruck | 0,5 |
| Vierter Abdruck | 0,8 |
| Fünfter Abdruck | 1 |
| Sechster Abdruck | 1,1 |
| Siebter Abdruck | 1,2 |

Nach einer sequenziellen Verwendung der Platten des vorgeschlagenen Satzes von Platten bei dem Säugling mit Lippen-Kiefer-Gaumen-Segelspalte verlaufen die Prämaxilla 12 und das Vomer 13 entlang der Abdruckmittelebene 19 nahezu mittig zwischen den Oberkiefersegmenten 14, 15. Die kugelartigen Ausformungen der Prämaxilla 12 sind dann üblicherweise beiderseits der Abdruckmittelebene 19 mit etwa gleich großen Abständen zu den lateralen Kiefersegmenten 14, 15 angeordnet. Durch eine Wiederherstellung einer gleichmäßigen Anordnung der Oberkieferelemente kann ein gleichmäßiges Zusammenwachsen dieser Oberkieferelemente gefördert und ein Auftreten einer Spaltenbildung verringert werden.

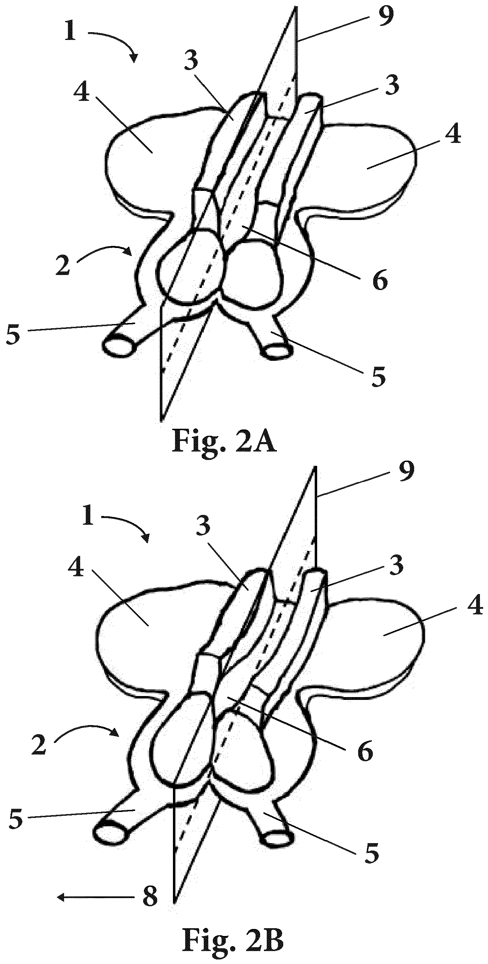

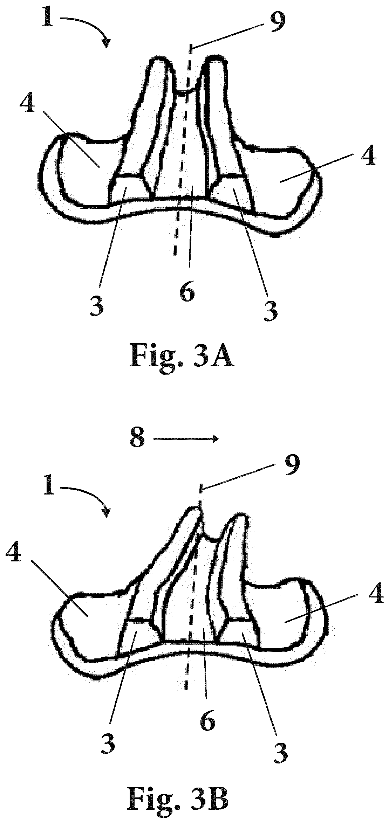

In den Figuren 2A und 3A ist ein Ausführungsbeispiel einer ersten Platte 1 zum Einsetzen in den Gaumen eines Säuglings mit Lippen-Kiefer-Gaumen-Segelspalte zum Begradigen der Prämaxilla 12 und des Vomers 13 des Säuglings und zum Verringern einer Fehlstellung der Prämaxilla 12 und des Vomers 13 des Säuglings des vorgeschlagenen Satzes von Platten dargestellt. Die Figur 2A zeigt die erste Platte 1 in der perspektivischen Vorderansicht. Die Figur 3A bildet hingegen die erste Platte 1 in der perspektivischen Hinteransicht ab. Die Platte 1 weist zwei beiderseits und entlang einer senkrecht zur Platte 1 gedachten Mittelebene 9 nebeneinander und plattenoberseitig verlaufende Stege 3 auf. Zwischen den Stegen 3 ist ein Freiraum 6 zum Aufnehmen des Vomers 13 und zum Begradigen des Vomers 13 ausgebildet. Die Stege 3 verlaufen von einem ersten Ende der Platte 1 parallel und geradlinig zur Mittelebene 9 in einem gleichmäßigen Abstand von beispielsweise 0,8 cm zueinander. Der Abstand kann aber auch beispielsweise mindestens 0,3 cm und beispielsweise höchstens 1,5 cm betragen. Darüber hinaus kann der Abstand auch ungleichmäßig sein und sich beispielsweise entlang der Mittelachse, insbesondere graduell, in einem Bereich von 0,3 cm bis 1,5 cm verändern. Eine Länge der Stege 3 entlang der Mittelebene 9 ist typischerweise größer als eine halbe Länge der Platte 1 entlang der Mittelachse 9, wobei die Länge der Platte 1 beispielsweise 5 cm beträgt. Eine Breite der Platte 1 quer zur Mittelachse 9 beträgt beispielsweise 4,5 cm. Die Mittelebene 9 der Platte 1 entspricht der Sagittalebene des Gaumens, wenn die Platte 1 in den Gaumen eingesetzt ist.

In der Tabelle 3 sind die Maße der Stege 3 und der Platten des vorgeschlagenen Satzes von Platten zur sequenziellen Verwendung angegeben. Beispielhaft sind sieben Platten aufgeführt. Die angegebenen Werte sind auf die Nachkommastelle gerundet. In Abhängigkeit von der anatomischen Gegebenheit des Gaumens des Säuglings kann es erforderlich sein, dass die Höhe der Stege 3 zwischen den verschiedenen Platten des Satzes von Platten variiert werden muss. Beispielsweise kann die Höhe der Stege 3 der ersten drei Platten jeweils zunehmen und ab der vierten Platte konstant bleiben. Eine anfänglich geringere Höhe der Stege 3 kann beispielsweise erforderlich sein, um die Nasenmuschel des Säuglings sukzessive zu verdrängen, bis die Stege 3 das Vomer 13 des Säuglings umschließen können. Die Länge der Platten des Satzes von Platten kann beispielsweise von der ersten zur zweiten Platten zunehmen, um das Vomer 13 des Säuglings möglichst großflächig durch die Stege 3 zu umgreifen, wodurch ein Aufrichten des Vomers 13 des Säuglings erleichtert werden kann. Ab beispielsweise der dritten Platte des Satzes von Platten kann die Länge der einzelnen Platten von Platte zu Platte fortlaufend abnehmen, da sich das Vomer 13 des Säuglings mit fortlaufender Plattenzahl vorzugsweise begradigt und eine geringere Fläche zum Umgreifen des Vomers 13 durch die Stege 3 notwendig sein kann. Durch eine fortlaufende Verringerung der Länge der Platten von Platte zu Platte des Satzes von Platten kann das Einsetzen der Platte in den Gaumen des Säuglings sowie das Tragefühl für den Säugling verbessert werden, ohne dabei die Funktion der Platte zu beeinträchtigen.

**Tabelle 3: Beispielhafte Maße der einzelnen Platten und deren Stege des vorgeschlagenen Satzes von Platten**

| Satz von Platten | Höhe der Stege in cm | Breite des ersten Stegs in cm | Breite des zweiten Stegs in cm | Breite der Platte in cm | Länge der Platte (ohne Halteeinrichtung) in cm |
|---|---|---|---|---|---|
| Erste Platte | 0,8 | 0,9 | 0,3 | 4,5 | 3,5 |
| Zweite Platte | 1 | 0,8 | 0,5 | 4,6 | 4 |
| Dritte Platte | 1,2 | 0,6 | 0,8 | 4,6 | 3,7 |
| Vierte Platte | 1,4 | 0,6 | 0,8 | 4,6 | 3,4 |
| Fünfte Platte | 1,4 | 0,6 | 0,7 | 4,6 | 3,2 |
| Sechste Platte | 1,4 | 0,6 | 0,7 | 4,6 | 3 |
| Siebte Platte | 1,4 | 0,6 | 0,7 | 4,6 | 2,8 |

Beim Einsetzen der Platte 1 in den Gaumen wird das Vomer 13 in den Freiraum 6 aufgenommen und zwischen den zwei Stegen 3 eingefasst. Die Form der Platte 1 ist derart an den Gaumen des Säuglings angepasst, dass sich die Platte 1 insbesondere durch die Flügel 4 am Gaumen abstützt und in mehreren Richtungen am Gaumen fixiert sitzt. Durch den festen Sitz der Platte 1 am Gaumen und einen Versatz des Verlaufs der Stege 3 zum gegenwärtigen Verlauf des Vomers 13 kann der Form des Verlaufs des in den Freiraum 6 eingefassten Vomers 13 mittels einer therapeutischen Kraft ein neuer Verlauf aufgezwungen werden. Als therapeutische Kraft wird die Kraft angesehen, die durch die vorgeschlagene Platte 1 auf den Gaumen des Säuglings wirkt, wenn die Platte 1 in den Gaumen des Säuglings eingesetzt ist.

Die Stege 3 der Platte 1 sind entlang der Mittelebene 9 geradlinig und entlang der Mittelebene 9 nebeneinander verlaufend ausgebildet. Geradlinig und nebeneinander verlaufende Stege 3 sind insbesondere von Vorteil, um solange eine therapeutische Kraft auf das Vomer 13 auszuüben, bis der Verlauf des Vomers 13 dem Verlauf der geradlinigen und entlang der Mittelebene 9 nebeneinander verlaufenden Stege 3 entspricht. Bei der Verwendung der in Fig. 2A und 3A dargestellten Platte 1 kann ein gekrümmter Verlauf des Vomers 13 durch eine therapeutische Kraft begradigt werden oder eine Krümmung des ungebogenen Vomers 13 verhindert werden. Durch ein nicht gekrümmtes Vomer 13 kann das Auftreten von Folgeschäden, insbesondere von Atmungsbehinderungen des Patienten im nasalen Bereich entscheidend reduziert werden.

Die Stege 3 haben ferner jeweils ein erstes Ende, welches in die gleiche Richtung des ersten Endes der Platte 1 zeigt. An einem dem ersten Ende der Stege 3 gegenüberliegenden zweiten Ende der Stege 3 ist eine Aufnahme 2 mit einer muldenartigen Vertiefung angeordnet. Die muldenartige Vertiefung weist zwei kugelförmige Auswölbungen auf und ist derart ausgebildet, dass eine Innenfläche der muldenartigen Vertiefung die Prämaxilla 12 umlaufend umschließen kann, wenn die Platte 1 in den Gaumen eingesetzt ist. Ein umlaufendes Umschließen der Prämaxilla 12 durch die muldenartige Vertiefung ermöglicht einen besonders vorteilhaften Halt der Prämaxilla, wenn die Platte in den Gaumen des Säuglings eingesetzt ist. Eine quer zur Mittelebene 9 bestimmte maximale Breite eines die Aufnahme 2 enthaltenden Aufnahmebereichs der Platte 1 beträgt 80 Prozent einer quer zur Mittelebene 9 bestimmten maximalen Breite der Platte 1. Aufgrund der Form und der Anordnung der Aufnahme 2 der in Fig. 2A dargestellten Platte 1 kann die Prämaxilla 12 mittels der therapeutischen kraft der Platte 1 in eine gerade Form und zu gleichen Teilen beiderseits der Mittelachse 9 angeordnet werden. Außerdem kann die Form und Anordnung der Prämaxilla 12 durch die Verwendung der Platte 1 beibehalten werden. Die in Fig. 2A gezeigte Platte 1 kann eine letzte Platte in einem Satz von Platten zur sequenziellen Behandlung sein, die für einen gewünschten Endzustand des Verlaufs des Vomers 13 und der Form und Anordnung der Prämaxilla 12 verwendet werden kann.

Ferner weist die Platte 1 zwei beiderseits der Mittelebene 9 verlaufende Flügel 4 auf, die derart ausgebildet sind, um wenigstens bereichsweise am Gaumen anzuliegen. Die Flügel 4 bilden mit ihren Oberseiten eine Kontaktfläche der Platte 1 mit dem Gaumen, wenn die Platte 1 in den Gaumen eingesetzt ist. Die Flügel 4 bewirken, dass die Platte 1 in einer vorgegebenen Position in den Gaumen des Säuglings einsetzbar ist. Außerdem stützen die Flügel 4 einen festen Sitz der Platte 1 im Gaumen des Säuglings, wenn die Platte 1 in den Gaumen eingesetzt ist.

Die Stege 3 sind über die Kontaktfläche erhaben und stehen senkrecht, d.h. in kranial, von der Kontaktfläche mit einer Höhe von beispielsweise 0,8 cm ab. Die Kontaktfläche stellt sicher, dass die Stege 3 nicht zu weit in kranial in den nasalen Bereich des Säuglings eindringen, um Atembehinderungen des Säuglings aufgrund der Stege 3 zu vermeiden, wenn die Platte 1 in den Gaumen eingesetzt ist.

Darüber hinaus sind die Flügel 4 an von der Mittelebene 9 abgewandten Seiten der Stege 3 angeordnet, sodass der jeweilige Flügel 4 an den jeweiligen Steg 3 heranreicht. Eine entlang der Mittelebene 9 bestimmte maximale Länge der Flügel 4 beträgt 80 Prozent einer entlang der Mittelebene 9 bestimmten Länge. Außerdem definiert die maximale Länge der Flügel 4 einen entlang der Mittelebene 9 ausgerichteten Längenabschnitt, sodass sich eine quer zur Mittelebene 9 bestimmte maximale Breite der Platte 1 in dem Längenabschnitt um 50 Prozent reduziert, wobei eine entlang der Mittelebene 9 bestimmte Länge des Längenabschnitts 15 Prozent einer entlang der Mittelebene 9 bestimmten maximalen Länge der Platte 1 beträgt. Dadurch bildet die Platte eine T-förmige Struktur aus.

Ferner weist die Platte eine Halteeinrichtung 5 zum Einspannen einer Spannvorrichtung zur Krafteinwirkung auf die Platte entlang der Mittelebene mit zwei Fixiervorsprüngen auf. Die Fixiervorsprünge sind beiderseits der Mittelebene 9 an einem von den Stegen 3 abgewandten und in eine Richtung entlang der Mittelebene 9 verlaufenden Ende der Aufnahme 2 angeordnet. Die Fixiervorsprünge stehen nach vorn, d.h. in anterior, von der Aufnahme 2 ab. Wenn die Platte 1 in den Gaumen des Säuglings eingesetzt ist, stehen die Fixiervorsprünge üblicherweise aus dem Mund des Säuglings hervor. An den hervorstehenden Fixiervorsprüngen können Gummibänder eingespannt sein, um eine Krafteinwirkung auf die Platte 1 entlang der Mittelebene 9 zu erzeugen, um die Prämaxilla 12 und das Vomer 13 entlang der Mittelebene 9 zu verschieben. Somit können durch die Platte 1 Fehlstellungen der Prämaxilla 12 und des Vomers 13 zusätzlich in anterior und in posterior behoben und eine Krümmung des Vomers 13 verhindert werden.

In den Figuren 2B und 3B ist ein Ausführungsbeispiel einer zweiten Platte 1 zum Einsetzen in den Gaumen eines Säuglings mit Lippen-Kiefer-Gaumen-Segelspalte zum Begradigen des Vomers 13 des Säuglings des vorgeschlagenen Satzes von Platten in der Vorderansicht abgebildet. Die Figur 2B stellt die zweite Platte 1 in der perspektivischen Vorderansicht dar. Die Figur 3B zeigt die zweite Platte 1 in der perspektivischen Hinteransicht. Die zweite Platte 1 umfasst im Wesentlichen die Merkmale von der in Figur 2A dargestellten ersten Platte 1 und sich insofern unterscheidet, als dass die zweite Platte 1 einen Versatz des Verlaufs der Stege 3 und der Form und Anordnung der Aufnahme 2 gegenüber dem Verlauf der Stege 3 und der Form und Anordnung der Aufnahme 2 der ersten Platte 1 aufweist.

Die in Fig. 2B dargestellte zweite Platte 1 eines Satzes von Platten zur sequenziellen Behandlung kann für einen Anfangs- oder Ausgangszustand eines Oberkiefers eines Säuglings mit Lippen-Kiefer-Gaumen-Segelspalte geeignet sein. Die zweiten Platte 1 kann eine erste Verschiebung des Verlaufs des Vomers 13 im Anfangszustand und der Form und Anordnung der Prämaxilla 12 im Anfangszustand bewirken, indem die zweite Platte einen ersten Querversatz des Verlaufs der Stege 3 und der Form und Anordnung der Aufnahme 2 gegenüber dem Verlauf des Vomers 13 und der Form und Anordnung der Prämaxilla 12 im Anfangszustand aufweist, und als erstes in den Gaumen des Säuglings eingesetzt werden. Nachdem der Verlauf der Stege 3 der zweiten Platte 1 dem Verlauf des Vomers 13 und die Form und Anordnung der Aufnahme 2 der Form und Anordnung der Prämaxilla 12 aufgezwängt wurde, kann die zweite Platte 1 durch eine weitere Platte 1 des Satzes von Platten ausgetauscht werden. Die weitere Platte 1 kann eine zweite Verschiebung des Verlaufs des Vomers 13 und der Form und Anordnung der Prämaxilla 12 bewirken, indem die weitere Platte 1 einen zweiten Querversatz des Verlaufs der Stege 3 und der Form und Anordnung der Aufnahme 2 gegenüber dem Verlauf des Vomers 13 und der Form und Anordnung der Prämaxilla 12 im Anfangszustand aufweist, wobei der zweite Querversatz größer als der erste Querversatz ist, und als zweites in den Gaumen des Säuglings eingesetzt werden. Die weitere Platte 1 kann ebenfalls durch eine erneute Platte 1 mit einem weiteren Querversatz des Verlaufs der Stege 3 und der Form und Anordnung der Aufnahme 2 gegenüber dem Verlauf des Vomers 13 und der Form und Anordnung der Prämaxilla 12 im Anfangszustand ausgetauscht werden, wobei der weitere Querversatz größer als der zweite Querversatz ist. Die Richtung der Querversätze wird durch einen Richtungspfeil 8 dargestellt. Ein derartiges sukzessives Austauschen der Platten des Satzes von Platten kann solange erfolgen bis der Verlauf des Vomers 13 und die Form und Anordnung der Prämaxilla 12 soweit verschoben sind, dass die letzte Platte 1 aus Fig. 2A eingesetzt werden kann und den letzten Behandlungsschritt zur Begradigung des Vomers 13 und der Prämaxilla 12 vollzogen werden kann.

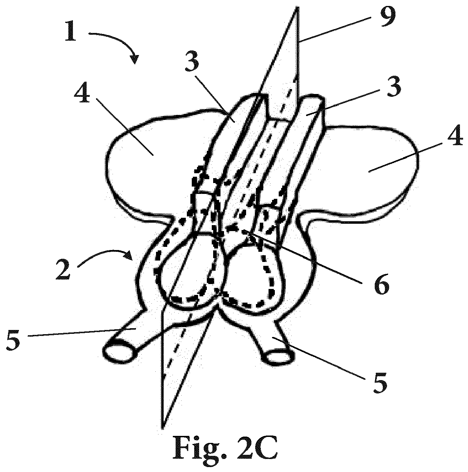

Die Fig. 2C zeigt eine perspektivische Darstellung der ersten Platte 1 aus Fig. 2A mit einer durch eine gestrichelte Kontur dargestellte Überlagerung des Verlaufs der Stege 3 und der Form und Anordnung der Aufnahme 2 der zweiten Platte 1 aus Fig. 2B des vorgeschlagenen Satzes von Platten in der Vorderansicht. Die Überlagerung macht deutlich, dass die zweite Platte 1 Querversätze des Verlaufs der zweiten Enden der Stege 3 und der Aufnahme 2 gegenüber den Verläufen der zweiten Enden der Stege 3 und der Aufnahme 2 der ersten Platte 1 aufweist. Analog zu den Querversätzen sind aber auch Längsversätze der Verläufe der zweiten Enden der Stege 3 und der Aufnahme 2 der zweiten Platte 1 Querversätze gegenüber den Verläufen der zweiten Enden der Stege 3 und der Aufnahme 2 der ersten Platte 1 möglich.

Typischerweise werden die Querversätze mittels aus dem Abdruck 10 des Oberkiefers des Säuglings mit Lippen-Kiefer-Gaumen-Segelspalte berechneten Fehlstellungen der Prämaxilla 12, des Vomers 13 und der lateralen Kiefersegmente 14, 15 bestimmt. Die Querversätze sind derart ausgebildet, dass beim Einsetzen der zweiten Platte 1 in den Gaumen des Säuglings durch die zweite Platte 1 eine therapeutische Kraft auf die Prämaxilla 12 und das Vomer 13 des Säuglings ausgeübt wird. Dies wird typischerweise dadurch erreicht, dass die Verläufe der Stege 3 der zweiten Platte 1 einen geringeren Querversatz aufweisen als ein Querversatz, der durch eine Fehlstellung der Prämaxilla 12 und des Vomers 13 gegeben ist. Die therapeutische Kraft sollte dabei vorteilhafterweise in eine entgegen der durch den Richtungspfeil 8 dargestellten Richtung ausgeübt werden, damit die Fehlstellungen der Prämaxilla 12 und des Vomers 13 durch die eingesetzte zweite Platte 1 verringert werden können. Durch ein Einsetzen der zweiten Platte 1 im Gaumen des Säugling mit Lippen-Kiefer-Spalte kann solange eine Krafteinwirkung auf die Prämaxilla 12 und das Vomer 13 erfolgen, bis diese sich derart verschieben, dass sie in die durch die Stege 3 und die Aufnahme der zweiten Platte 1 vorgegebene Form angeordnet sind. Nun kann die in den Gaumen eingesetzte zweite Platte 1 durch die erste Platte 1 ausgetauscht werden, um eine erneute Krafteinwirkung auf die verschobene Prämaxilla 12 und das verschobene Vomer 13 bereitzustellen. Durch die in den Gaumen eingesetzte erste Platte 1 verschieben sich die Prämaxilla 12 und das Vomer 13 erneut, bis sie die durch die erste Platte 1 vorgegebene Form eingenommen haben. Die Prämaxilla 12 und das Vomer 13 sind dann in einer Position mittig zwischen den Oberkiefersegmenten 14, 15 geradlinig entlang der Mittelebene 9 positioniert. Somit können die Fehlstellungen der Prämaxilla 12 und des Vomers 13 mit einer derartigen sequenziellen Behandlung durch den Satz von Platten behoben werden und dabei eine Krümmung des Vomers 13 vermieden werden.

Je nach Schwere der Fehlstellung können weitere Platten 1 aus dem Satz von Platten vorgesehen sein, die nacheinander in den Gaumen des Säuglings eingesetzt werden und die Fehlstellung der Prämaxilla 12 sowie des Vomers 13 sequenziell verringern. Dabei ist bei der Herstellung weiterer Platten zu berücksichtigen, dass das Volumen der Oberkieferelemente mit im Laufe der Zeit zunimmt und die weiteren Platten an die neuen Volumina angepasst werden müssen.

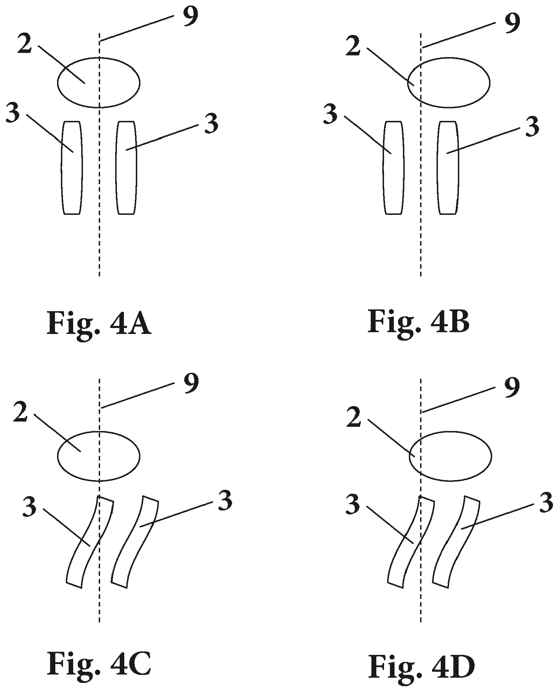

In den Fign. 4A-D sind verschiedene Anordnungen der Aufnahme 2 und der Stege 3 gegenüber der Mittelebene 9 einer Platte aus einem Satz von Platten schematisch dargestellt.

Die in Fig. 4A gezeigte Anordnung der Aufnahme 2 und der Stege 3 entspricht einer Anordnung der Aufnahme 2 und der Stege 3 der ersten Platte 1 aus Fig. 2A. Die Stege 3 sind geradlinig entlang der Mittelebene 9 angeordnet und die Aufnahme 2 ist zu gleichen Teilen beidseitig der Mittelebene 9 angeordnet.

Es kann aber auch vorgesehen sein, wie in Fig. 4B und 4C gezeigt, dass entweder die Aufnahme 2 einen Querversatz quer zur Mittelebene 9, beispielsweise gegenüber der in Fig. 4A gezeigten Anordnung der Aufnahme 2, oder der Verlauf der Stege einen Querversatz quer zur Mittelebene 9, beispielsweise gegenüber der in Fig. 4A gezeigten Anordnung der Stege 3, aufweist. Auf diese Weise können der Verlauf des Vomers 13 beibehalten sowie die Form und Anordnung der Prämaxilla 12 verändert werden oder der Verlauf des Vomers 13 verändert sowie die Form und Anordnung der Prämaxilla 12 beibehalten werden. Je nach Form und Anordnung der Aufnahme 2 und dem Verlauf der Stege 3 können somit mit dem Satz von Platten verschiedene Fehlstellungen des Vomers 13 und der Prämaxilla 12 unabhängig voneinander und gezielt behandelt werden.

Die in Fig. 4D gezeigte Anordnung der Aufnahme 2 und der Stege 3 entspricht einer Anordnung der Aufnahme 2 und der Stege 3 der zweiten Platte 1 aus Fig. 2B. Die Anordnung der Aufnahme 2 und der Verlauf der Stege 3 weisen einen Querversatz quer zur Mittelebene 9 beispielsweise gegenüber der in Fig. 4A gezeigten Anordnung der Aufnahme 2 und der Stege 3 auf. Auf diese Weise können der Verlauf des Vomers 13 und die Anordnung der Prämaxilla 12 gleichzeitig verändert werden.

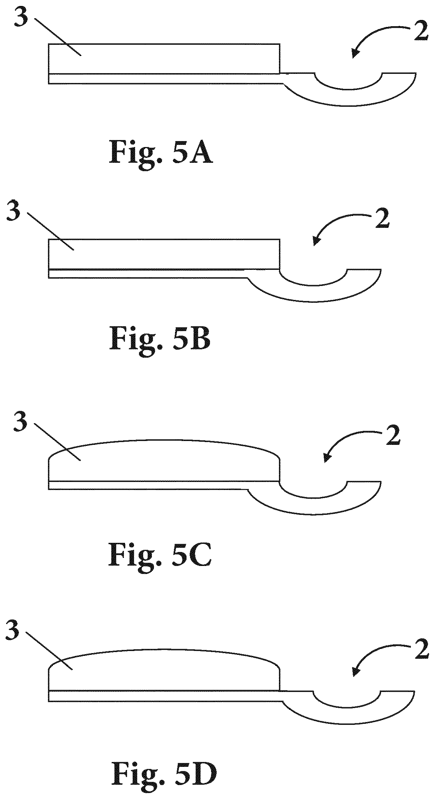

In den Fign. 5A-D sind Querschnitte entlang der Mittelebene 9 von verschiedenen Platten eines Satzes von Platten dargestellt. Die Platten weisen jeweils einen Übergang des Stegs 3 zu der Aufnahme 2 auf. Fig. 5A zeigt einen rechteckigen Steg 3 an, wobei zwischen der muldenartigen Vertiefung der Aufnahme 2 und dem Steg 3 ein Abstand mit einem geraden Plateau verbleibt. Die in Fig. 5B gezeigte Platte weist ebenfalls einen rechteckigen Steg 3 auf, wobei ein unmittelbarer Übergang zwischen dem Steg 3 und der muldenartigen Vertiefung der Aufnahme 2 besteht.

Fig. 5C zeigt hingegen einen abgerundeten Steg 3 mit einem Abstand zwischen der muldenartigen Vertiefung der Aufnahme 2 und dem Steg 3 ebenfalls mit einem geraden Plateau an. Die in Fig. 5D dargestellte Platte weist ebenfalls einen abgerundeten Steg 3 auf, wobei ein unmittelbarer Übergang zwischen dem Steg 3 und der muldenartigen Vertiefung der Aufnahme 2 besteht. Je nach anatomischer Gegebenheit des Gaumens des Säuglings wie beispielsweise die Nasenmuschel können das Ende des Steges 3 und/oder der Übergang zwischen Steg 3 und der muldenartigen Vertiefung der Aufnahme 2 an die anatomische Gegebenheit des Gaumens des Säuglings angepasst sein. Diese Anpassung sowie die abgerundeten Stege 3 können dabei helfen, den Schmerz für den Säugling zu reduzieren, wenn die Platte in den Gaumen des Säuglings eingesetzt ist.

## Patentansprüche

1. Platte (1) zum Einsetzen in den Gaumen, wobei die Platte (1) beiderseits einer senkrecht zur Platte (1) gedachten Mittelebene (9) verlaufende Flügel (4) aufweist, die derart ausgebildet sind, um wenigstens bereichsweise am Gaumen anzuliegen,
**gekennzeichnet durch**
zwei Stege (3), die beiderseits der Mittelebene (9) verlaufen, so dass ein zwischen den Stegen (3) verlaufender Freiraum (6) zum Aufnehmen des Vomers (13) ausgebildet ist.

2. Platte (1) nach Anspruch 1, **gekennzeichnet durch** eine Aufnahme (2) zum Aufnehmen der Prämaxilla (12), wobei die Aufnahme (2) wenigstens eine muldenartige Vertiefung umfasst.

3. Platte (1) nach Anspruch 2, **dadurch gekennzeichnet, dass** die Aufnahme (2) an einem in eine Richtung entlang der Mittelebene (9) verlaufenden Ende einer der Stege (3) angeordnet ist und dass die muldenartige Vertiefung derart ausgebildet ist, dass eine Innenfläche der muldenartigen Vertiefung die Prämaxilla (12) umlaufend umschließt, wenn die Platte (1) in den Gaumen eingesetzt ist.

4. Platte (1) nach einem der Ansprüche 2 oder 3, **dadurch gekennzeichnet, dass** eine quer zur Mittelebene (9) bestimmte maximale Breite eines die Aufnahme (2) enthaltenden Aufnahmebereichs der Platte (1) höchstens 80 Prozent, vorzugsweise höchstens 70 Prozent, besonders bevorzugt höchstens 60 Prozent einer quer zur Mittelebene (9) bestimmten maximalen Breite der Platte (1) beträgt.

5. Platte (1) nach einem der vorhergehenden Ansprüche, **dadurch gekennzeichnet, dass** die Flügel (4) eine Kontaktfläche der Platte (1) mit dem Gaumen aufweisen und dass die Stege (3) derart an einer Seite der Platte abstehen, dass sie über die Kontaktfläche erhaben sind.

6. Platte (1) nach Anspruch 5, **dadurch gekennzeichnet, dass** eine senkrecht zur Kontaktfläche und ausgehend von der Kontaktfläche bestimmte Höhe der Stege (3) mindestens 0,4 cm, vorzugsweise mindestens 0,8 cm, besonders bevorzugt mindestens 1,2 cm und/oder höchstens 2 cm, vorzugsweise höchstens 1,8 cm, besonders bevorzugt höchstens 1,5 cm beträgt.

7. Platte (1) nach einem der vorhergehenden Ansprüche, **dadurch gekennzeichnet, dass** die Stege (3) quer zur Mittelebene (9) voneinander beabstandet sind, wobei ein minimaler Abstand der Stege (3) voneinander mindestens 0,3 cm, vorzugsweise mindestens 0,5 cm, besonders bevorzugt mindestens 0,7 cm und/oder höchstens 2 cm, vorzugsweise höchstens 1,5 cm, besonders bevorzugt höchstens 1 cm beträgt.

8. Platte (1) nach einem der vorhergehenden Ansprüche, **dadurch gekennzeichnet, dass** die Flügel (4) an von der Mittelebene (9) abgewandten Seiten der Stege (3) angeordnet sind.

9. Platte (1) nach Anspruch 8, **dadurch gekennzeichnet, dass** eine entlang der Mittelebene (9) bestimmte maximale Länge der Flügel (4) höchstens 100 Prozent, vorzugsweise höchstens 90 Prozent, besonders bevorzugt höchstens 80 Prozent einer entlang der Mittelebene (9) bestimmten Länge einer der Stege (3) beträgt.

10. Platte (1) nach Anspruch 9, **dadurch gekennzeichnet, dass** die maximale Länge der Flügel (4) einen entlang der Mittelebene (9) ausgerichteten Längenabschnitt definiert und dass sich eine quer zur Mittelebene (9) bestimmte maximale Breite der Platte (1) in dem Längenabschnitt um mindestens 40 Prozent, vorzugsweise um mindestens 50 Prozent, besonders bevorzugt um mindestens 60 Prozent reduziert, wobei eine entlang der Mittelebene (9) bestimmte Länge des Längenabschnitts höchstens 25 Prozent, vorzugsweise höchstens 15 Prozent, besonders bevorzugt höchstens zehn Prozent einer entlang der Mittelebene (9) bestimmten maximalen Länge der Platte (1) beträgt.

11. Platte (1) nach einem der Ansprüche 2-4 oder nach einem der Ansprüche 3-10, soweit rückbezogen auf einen der Ansprüche 2-4, **gekennzeichnet durch** eine Halteeinrichtung (5) zum Einspannen einer Spannvorrichtung zur Krafteinwirkung auf die Platte (1) entlang der Mittelebene (9), wobei die Halteeinrichtung (5) an einem von den Stegen (3) abgewandten und in eine Richtung entlang der Mittelebene (9) verlaufenden Ende der Aufnahme (2) angeordnet ist.

12. Satz von Platten zum sequenziellen Begradigen des Vomers (13) eines Säuglings mit Lippen-Kiefer-Gaumen-Segelspalte, umfassend:
mindestens eine erste Platte nach einem der vorhergehenden Ansprüche und eine zweite Platte nach einem der vorhergehenden Ansprüche, wobei sich ein erster räumlicher Verlauf der Stege der ersten Platte in eine Richtung entlang der Mittelebene (9) der ersten Platte von einem zweiten Verlauf der Stege der zweiten Platte in eine Richtung entlang der Mittelebene (9) der zweiten Platte voneinander unterscheidet.

13. Satz von Platten nach Anspruch 12,
wobei die Stege (3) der ersten Platte einen ersten und einen zweiten Steg umfassen,
wobei die Stege (3) der zweiten Platte einen ersten und einen zweiten Steg umfassen,
wobei jeder der Stege jeweils ein in eine Richtung entlang der Mittelebene (9) verlaufendes erstes Ende und ein dem ersten Ende gegenüberliegendes zweites Ende aufweist,
wobei das erste Ende des ersten Stegs der ersten Platte gegenüber dem zweiten Ende des ersten Stegs der ersten Platte quer zur Mittelebene (9) der ersten Platte um einen ersten Querversatz versetzt angeordnet ist,
wobei das erste Ende des zweiten Stegs der ersten Platte gegenüber dem zweiten Ende des zweiten Stegs der ersten Platte quer zur Mittelebene (9) der ersten Platte um einen zweiten Querversatz versetzt angeordnet ist,
wobei das erste Ende des ersten Stegs der zweiten Platte gegenüber dem zweiten Ende des ersten Stegs der zweiten Platte quer zur Mittelebene (9) der zweiten Platte um einen dritten Querversatz versetzt angeordnet ist,
wobei das erste Ende des zweiten Stegs der zweiten Platte gegenüber dem zweiten Ende des zweiten Stegs der zweiten Platte quer zur Mittelebene (9) der zweiten Platte um einen vierten Querversatz versetzt angeordnet ist,
wobei der erste Querversatz um mindestens 30 Prozent, vorzugsweise um mindestens zehn Prozent, besonders bevorzugt um mindestens fünf Prozent kleiner ist als der dritte Querversatz und/oder
wobei der zweite Querversatz um mindestens 20 Prozent, vorzugsweise um mindestens zehn Prozent, besonders bevorzugt um mindestens fünf Prozent kleiner ist als der vierte Querversatz; und/oder
wobei das erste Ende des ersten Stegs der ersten Platte gegenüber dem zweiten Ende des ersten Stegs der ersten Platte entlang einer Richtung parallel zur Mittelebene (9) der ersten Platte um einen ersten Längsversatz versetzt angeordnet ist,
wobei das erste Ende des zweiten Stegs der ersten Platte gegenüber dem zweiten Ende des zweiten Stegs der ersten Platte entlang einer Richtung parallel zur der Mittelebene (9) der ersten Platte um einen zweiten Längsversatz versetzt angeordnet ist,
wobei das erste Ende des ersten Stegs der zweiten Platte gegenüber dem zweiten Ende des ersten Stegs der zweiten Platte entlang einer Richtung parallel zur der Mittelebene (9) der zweiten Platte um einen dritten Längsversatz versetzt angeordnet ist,
wobei das erste Ende des zweiten Stegs der zweiten Platte gegenüber dem zweiten Ende des zweiten Stegs der zweiten Platte entlang einer Richtung parallel zur der Mittelebene (9) der zweiten Platte um einen vierten Längsversatz versetzt angeordnet ist,
wobei der erste Längsversatz um mindestens 15 Prozent, vorzugsweise um mindestens zehn Prozent, besonders bevorzugt um mindestens fünf Prozent kleiner ist als der dritte Längsversatz und/oder
wobei der zweite Längsversatz um mindestens 15 Prozent, vorzugsweise um mindestens zehn Prozent, besonders bevorzugt um mindestens fünf Prozent kleiner ist als der vierte Längsversatz.

14. Verfahren zum Herstellen einer Platte (1) zum Einsetzen in den Gaumen eines Säuglings mit Lippen-Kiefer-Gaumen-Segelspalte zum Begradigen des Vomers (13) des Säuglings, umfassend:
Herstellen und Ausmessen eines Abdrucks (10) des Oberkiefers des Säuglings zum Bestimmen einer räumlichen Anordnung des Vomers (13), der Prämaxilla (12) und der beiden lateralen Oberkiefersegmente (14, 15) des Säuglings relativ zueinander,
Bestimmen einer quer zur Sagittalebene (19) des Säuglings bestimmten Vomerquerfehlstellung des zur Prämaxilla (12) gerichteten Endes des Vomers (13) und/oder
Bestimmen einer entlang der Sagittalebene (19) des Säuglings bestimmten Vomerlängsfehlstellung des zur Prämaxilla (12) gerichteten Endes des Vomers (13), und
Herstellen einer Platte (1) nach einem der Ansprüche 1 bis 11, wobei die Stege (3) der Platte (1) als ein erster Steg und als ein zweiter Steg mit jeweils einem in eine Richtung entlang der Mittelebene (9) verlaufenden ersten Ende und einem dem ersten Ende gegenüberliegenden zweiten Ende derart ausgebildet werden, dass
(i) das erste Ende des ersten Stegs gegenüber dem zweiten Ende des ersten Stegs um einen quer zur Mittelebene (9) der Platte (1) bestimmten ersten Querversatz versetzt angeordnet ist und dass das erste Ende des zweiten Stegs gegenüber dem zweiten Ende des zweiten Stegs um einen zur Mittelebene (9) der Platte (1) bestimmten zweiten Querversatz versetzt angeordnet ist,
wobei der erste Querversatz und der zweite Querversatz jeweils abhängig von der zuvor bestimmten Vomerquerfehlstellung gewählt wird; und/oder
(ii) das erste Ende des ersten Stegs gegenüber dem zweiten Ende des ersten Stegs um einen entlang einer Richtung parallel zur Mittelebene (9) der Platte (1) bestimmten ersten Längsversatz versetzt angeordnet ist und dass das erste Ende des zweiten Stegs gegenüber dem zweiten Ende des zweiten Stegs um einen entlang einer Richtung parallel zur Mittelebene (9) der Platte (1) bestimmten zweiten Längsversatz versetzt angeordnet ist,
wobei der erste Längsversatz und der zweite Längsversatz jeweils abhängig von der zuvor bestimmten Vomerlängsfehlstellung gewählt wird.

15. Verfahren zum Herstellen eines Satzes von Platten zum Einsetzen in den Gaumen eines Säuglings mit Lippen-Kiefer-Gaumen-Segelspalte zum Begradigen des Vomers (13) des Säuglings, umfassend:
Herstellen einer ersten Platte nach Anspruch 14 und einer zweiten Platten nach Anspruch 14,
wobei der erste Querversatz des ersten Endes des ersten Stegs der ersten Platte kleiner gewählt wird als der erste Querversatz des ersten Endes des ersten Stegs der zweiten Platte und wobei der zweite Querversatz des ersten Endes des zweiten Stegs der ersten Platte kleiner gewählt wird als der zweite Querversatz des ersten Endes des zweiten Stegs der zweiten Platte; und/oder
wobei der erste Längsversatz des ersten Endes des ersten Stegs der ersten Platte kleiner gewählt wird als der erste Längsversatz des ersten Endes des ersten Stegs der zweiten Platte und wobei der zweite Längsversatz des ersten Endes des zweiten Stegs der ersten Platte kleiner gewählt wird als der zweite Längsversatz des ersten Endes des zweiten Stegs der zweiten Platte.
